# EUROPEAN PATENT SPECIFICATION

(11) **EP 2 258 720 B1**
(45) Date of publication and mention of the grant of the patent: **05.09.2018**
(21) Application number: 10163251.1
(22) Date of filing: 19.05.2010
(51) Int. Cl.: C07K 14/705, C12N 15/62, A61K 38/17, A61P 35/00

(54) **MULTIPLE TARGET T CELL RECEPTOR**
MEHRFACHZIEL-T-ZELLEN-REZEPTOR
RÉCEPTEUR DE LYMPHOCYTE T À CIBLES MULTIPLES

(30) Priority: 19.05.2009 EP 09160679
(43) Date of publication of application: 08.12.2010
(73) Proprietor: Max Delbrück Centrum für Molekulare Medizin (MDC) Berlin-Buch;, 13125 Berlin (DE)
(72) Inventor: Kieback, Elisa, 10405 Berlin (DE); Charo, Jehad, 10115 Berlin (DE); Blankenstein, Thomas, 13467 Berlin (DE); Uckert, Wolfgang, 13125 Berlin (DE); Perez, Cynthia, 13187 Berlin (DE)
(74) Representative: Isarpatent

(56) References cited:
- EP-A- 1 878 744
- WO-A-01/62908
- WO-A-01/93913
- WO-A-2008/143794
- WO-A1-2009/117117
- US-A1- 2003 144 474
- US-A1- 2004 253 632
- US-A1- 2005 214 284
- US-B1- 6 534 633
- US-B1- 6 825 325
- ARAP W ET AL: "Cancer treatment by targeted drug delivery to tumor vasculature in a mouse model." SCIENCE (NEW YORK, N.Y.) 16 JAN 1998, vol. 279, no. 5349, 16 January 1998 (1998-01-16), pages 377-380, XP002550364 ISSN: 0036-8075
- KIEBACK ELISA ET AL: "A safeguard eliminates T cell receptor gene-modified autoreactive T cells after adoptive transfer." PROCEEDINGS OF THE NATIONAL ACADEMY OF SCIENCES OF THE UNITED STATES OF AMERICA 15 JAN 2008, vol. 105, no. 2, 15 January 2008 (2008-01-15), pages 623-628, XP002550365 ISSN: 1091-6490
- MATTHIAS LEISEGANG ET AL: "Enhanced functionality of T cell receptor-redirected T cells is defined by the transgene cassette" JOURNAL OF MOLECULAR MEDICINE, SPRINGER, BERLIN, DE, vol. 86, no. 5, 12 March 2008 (2008-03-12) , pages 573-583, XP019608780 ISSN: 1432-1440
- SOMMERMEYER DANIEL ET AL: "Designer T cells by T cell receptor replacement." EUROPEAN JOURNAL OF IMMUNOLOGY NOV 2006, vol. 36, no. 11, November 2006 (2006-11), pages 3052-3059, XP002550367 ISSN: 0014-2980
- NOLAN K F ET AL: "Bypassing immunization: optimized design of "designer T cells" against carcinoembryonic antigen (CEA)-expressing tumors, and lack of suppression by soluble CEA." CLINICAL CANCER RESEARCH : AN OFFICIAL JOURNAL OF THE AMERICAN ASSOCIATION FOR CANCER RESEARCH DEC 1999, vol. 5, no. 12, December 1999 (1999-12), pages 3928-3941, XP002550366 ISSN: 1078-0432
- MOSQUERA LUIS A ET AL: "In vitro and in vivo characterization of a novel antibody-like single-chain TCR human IgG1 fusion protein" JOURNAL OF IMMUNOLOGY, AMERICAN ASSOCIATION OF IMMUNOLOGISTS, US, vol. 174, no. 7, 1 April 2005 (2005-04-01) , pages 4381-4388, XP002504087 ISSN: 0022-1767
- KAMMERTOENS THOMAS ET AL: "Making and circumventing tolerance to cancer." EUROPEAN JOURNAL OF IMMUNOLOGY SEP 2009 LNKD- PUBMED:19634191, vol. 39, no. 9, September 2009 (2009-09), pages 2345-2353, XP002598509 ISSN: 1521-4141
- PINTO SHEENA ET AL: "Misinitiation of intrathymic MART-1 transcription and biased TCR usage explain the high frequency of MART-1-specific T cells", EUROPEAN JOURNAL OF IMMUNOLOGY, vol. 44, no. 9, September 2014 (2014-09), pages 2811-2821, ISSN: 0014-2980(print)

### SEQUENCE LISTING

<110> Max-Delbrück-Centrum für Molekulare Medizin (MDC)
<120> Multiple target T cell receptor
<130> P26813-EP
<150> EP09160679.8
   <151> 2009-05-19
<160> 4
<170> PatentIn version 3.3
<210> 1
   <211> 6
   <212> PRT
   <213> artificial
<220>
   <223> peptide MARS
<400> 1
   Met Ala Arg Ser Gly Leu
   1               5
<210> 2
   <211> 9
   <212> PRT
   <213> artificial
<220>
   <223> peptide RGD4C
<400> 2
   Cys Asp Cys Arg Gly Asp Cys Phe Cys
   1               5
<210> 3
   <211> 10
   <212> PRT
   <213> artificial
<220>
   <223> myc-tag
<400> 3
   Glu Gln Lys Leu Ile Ser Glu Glu Asp Leu
   1               5                   10
<210> 4
   <211> 20
   <212> PRT
   <213> artificial
<220>
   <223> 2xmyc-tag
<400> 4
   Glu Gln Lys Leu Ile Ser Glu Glu Asp Leu Glu Gln Lys Leu Ile Ser
   1               5                   10                  15
   Glu Glu Asp Leu
               20

## Description

### FIELD OF THE INVENTION

The present invention is directed to a functional T cell receptor (TCR) fusion protein (TFP) recognizing and binding to at least one MHC-presented epitope, and containing at least one amino acid sequence recognizing and binding an antigen. The present invention is further directed to an isolated nucleic acid molecule encoding the same, a T cell expressing said TFP, and a pharmaceutical composition for use in the treatment of diseases involving malignant cells expressing said tumor-associated antigen.

### BACKGROUND OF THE INVENTION

TCR's are members of the immunoglobulin superfamily and usually consist of two subunits, namely the α- and β-subunits. These possess one N-terminal immunoglobulin (Ig)-variable (V) domain, one Ig-constant (C) domain, a transmembrane/cell membrane-spanning region, and a short cytoplasmic tail at the C-terminal end. The variable domains of both the TCR α-chain and β-chain have three hypervariable or complementarity determining regions (CDRs), whereas the variable region of the β-chain has an additional area of hypervariability (HV4) that does not normally contact the MHC-peptide complex and therefore is not considered a CDR.

CDR3 is the main CDR responsible for recognizing processed antigen, although CDR1 of the alpha chain has also been shown to interact with the N-terminal part of the antigenic peptide, whereas CDR1 of the β-chain interacts with the C-terminal part of the peptide. CDR2 is thought to recognize the MHC. HV4 of the β-chain is not thought to participate in MHC-peptide complex recognition, but has been shown to interact with superantigens. The constant domain of the TCR domain consists of short connecting sequences in which a cysteine residue forms disulfide bonds, which forms a link between the two chains.

The affinity of TCR's for a specific antigen makes them valuable for several therapeutic approaches. For example, cancer patients, such as melanoma patients, can be effectively treated by using adoptive immunotherapy. That is to say, significant tumor regression can occur following adoptive transfer of T cells with anti-tumor specificity. However, patient-derived T cells may have sub-optimal activity. Therefore, there is current interest in using pre-characterized TCR genes to create designer lymphocytes for adoptive cell therapies.

The first clinical trials using adoptive transfer of TCR-transgenic T cells showed some clinical efficacy. However, the problem appears that therapies might fail due to so called antigen loss variants developed by cancer cells. Antigen- or MHC-loss variants are frequent mechanisms used by cancer cells that lead to their escape from detection and elimination by T cells. Whether it is due to active immune selection, as demonstrated by immunotherapy studies or due to genomic instability, required to maintain tumor outgrowth and the acquisition of its fully malignant phenotype, these escape mechanisms are very heterogeneous. They include mutations that lead to decreased or no MHC expression such as mutations in the MHC molecule heavy chains encoding genes or in those encoding for molecules involved in antigen processing and presentation including transporter associated with antigen presentation (TAP) and the light chain of MHC-I molecule; β2-microglobulin.

Altered level of expression of MHC can also be due to an effect mediated by suppressive cytokines such as interleukin 10. Recognition of tumor associated antigens can also be lost due to mutations or decreased expression level of these antigens. This was shown for melanoma antigens as well as for carcinomas and in an experimental mouse model and was associated with immune ignorance.

Another suggested mechanism for tumor escape from immune recognition is based on immunodominance. This mechanism favours sequential escape and selection of cancer cells expressing subdominant or cryptic antigen loss variants that can not be recognised by the mislead immune system.

The development of antigen loss variants represents a serious challenge for any successful immunotherapy approach, which is often based on targeting a single antigen or even an epitope (Leen et al. 2007). Yet, each single cancer cell expresses several antigens and 6-12 different MHC class I and II alleles. Studies of tumor-specific and -associated antigens have led to the isolation of many potential immunotherapy targets (Wang et al. 2006). With this myriad of targets, the earnest is on the ability of using these targets to kill tumor cells via the utilisation of their counter receptors.

Therapeutic interventions based on using monoclonal antibodies or the transfer of T cells or natural killer (NK) cells engineered with T cell receptor (TCR) or chimeric receptor genes represent a promising advance in immunotherapy (Leen et al. 2007). Several tumor-recognising antibodies and TCRs were recently isolated and used in experimental studies and in clinical trials and the list is continuously growing. Furthermore, some antibodies have already passed the scrutiny of regulatory bodies and are being currently used as a standard therapy. These include antibodies targeting angiogenesis, which is an essential process for solid tumor formation and survival.

Alternatively, targeting tumors with short peptide sequences derived from phage display or synthetic peptide library represents a promising approach in biotherapy (Arap et al. 1998). Among others, peptides specific for breast, prostate and colon carcinomas as well as those specific for neo-vasculatures were successfully isolated.

WO 01/62908 discloses a TCR fusion protein comprising a TCR recognition element and an immunoglobulin recognition element. However, there is no information contained regarding a TCR fusion protein comprising more than 2 functionalities and regarding the insertion sites suitable for introducing those functionalities in the TCR.

US 6,534,633 B1 discloses a binding molecule including a sc-TCR and, covalently linked through a peptide linker, a single chain antibody. Furthermore, the fusion protein described therein is soluble, i.e. not membrane-bound. There is no information contained that a binding peptide should be inserted into the TCR and, further, no information regarding the insertion sites suitable for introducing the same into the TCR is provided.

WO 01/93913 shows soluble T cell receptor fusion proteins of bispecific nature. Also here, the TCR and the biologically active polypeptide are connected by a peptide linker. There is no information contained that a binding peptide can be inserted into the TCR and regarding the insertion sites suitable for introducing the same into the TCR.

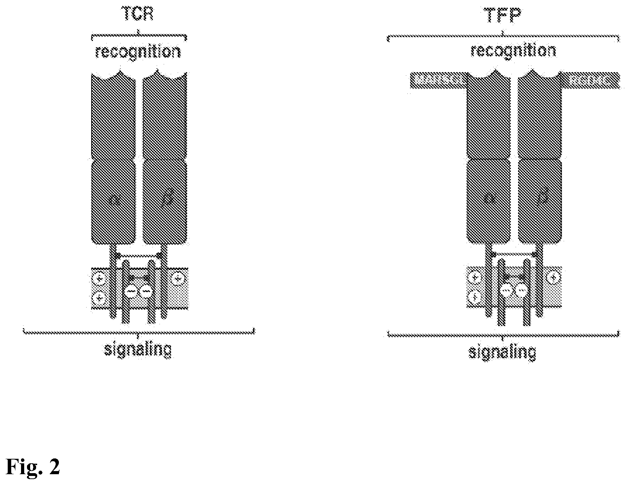

US 2004/253632 is concerned with compounds that modulate interactions between a T cell receptor (TCR) and a major histocompatibility complex (MHC) antigen (see p. 1, left col., para. [0001]). Recombinant TCR molecules are provided that include CD molecule sequences, e.g. a mammalian CD3ζ sequence (see e.g. p. 5, left col., para. [0057], FIG. 10). These TCR- CD3ζ fusion proteins are used in methods for detecting such compounds (see p. 19, left col., para. [0215]). According to p. 20, left col., para. [0220], the co-expression of the TCR molecule with the CD protein(s) allows the cells to be more sensitive to simulation by the MHC antigen molecule. FIG. 2 shows how the TCR:MHC antigen interaction may be assisted by another receptor such as a CD molecule. Example 11 discloses a screening assay based on the a scTCR-CD3ζ fusion protein.

Both experimental data and theoretical considerations suggest that the most effective approach of treating cancer would be through a vigorous immune response that can prevent the development of loss variants and enables the eradication of a large established tumor. This was most clearly established by the studies reported from H. Schreiber's lab, wherein successful T-cell immunotherapy was shown to operate at established tumors only when this tumor expressed a high level of the antigen, in a process that required recognition of the tumor stroma. Conversely, tumors established from the very same cell line but have a low level of the antigen expressed escaped the immune destruction and allowed the development of antigen-loss variant.

Therefore, new therapies are needed in order to address the above mentioned drawbacks of known therapeutic approaches. These new therapies should provide a vigorous immune response that can prevent the development of loss variants and should enable the eradication of a large established tumor.

### SUMMARY OF THE INVENTION

Therefore, it is an object of the present invention to provide a new therapeutic approach which allows an improved therapy of several diseases, such as tumor related diseases, autoimmune, hereditary, or infectious diseases, immunodeficiency, allergy and which might find application in transplantation.

It is a further object of the present invention to provide a functional TCR fusion protein (TFP), which shows high affinity against tumor-associated antigens and MHC-presented epitopes. It is a still further object of the invention to provide pharmaceutical compositions for use in adoptive cell therapy which allow an effective treatment of the diseases and conditions outlined above. In particular, it is an object of the invention to provide a functional TFP, which can overcome the problems associated with tumor escape variants due to antigen loss or major histocompatibility complex protein loss and which may target neo-vasculature-specific antigens at the same time.

These objects are achieved by the subject-matter of the independent claims. Preferred embodiments are indicated in the dependent claims.

Herein, a new type of therapy is disclosed that will target simultaneously at least two different antigens and may further target angiogenesis as well. This is exemplified by targeted antigens such as HER-2, a cell surface molecule that is presented independently of MHC restriction and the MHC-presented epitopes gp100₂₀₉ and gp33 derived from the melanoma associated antigen gp100 or the model tumor antigen gp1 from the lymphocytic choriomeningitis virus, respectively. Angiogenesis may, for example, be targeted through the integrins αvβ3 and its homologue αvβ5 expressed on tumor neo-vasculatures (Arap et al. 1998).

The inventors surprisingly could show that a trifunctional TCR fusion protein having at least three functionalities, i.e. one for a MHC-presented epitope, and at least two for an additional antigen, can be generated without affecting the original specificity for the MHC-complex.

Therefore, the TCR of the invention and the therapeutic approach disclosed herein enables multiple targeting of tumors via one genetic modification using one TCR. This is achieved by providing a TCR fusion protein which allows peptide guided and TCR guided attack of cells, in particular tumor cells, at the same time. Thus, the TCR fusion protein as disclosed herein will allow a remarkable improvement of therapies relying on adoptive T cell transfer.

### DETAILED DESCRIPTION OF THE INVENTION

According to a first aspect, the present invention provides a functional TCR α and/or β chain fusion protein recognizing and binding to at least one MHC-presented epitope, and containing at least two amino acid sequences recognizing and binding an antigen,
wherein the epitope is a tumor cell epitope and the antigen is a tumor cell antigen, and wherein the TCR fusion protein is trifunctional, where the first function is the function of the TCR α and/or β chain fusion protein to recognize and bind to at least one MHC-presented epitope; the second and third are based on amino acid sequences recognizing and binding an antigen, and
wherein
i) the at least two antigen binding amino acid sequences are added to the N-terminus of the α and/or β chain,
ii) the at least two antigen binding amino acid sequences are inserted into a constant or a variable region of said α and/or β chain, and/or
iii) the at least two antigen binding amino acid sequences are replacing a number of amino acids in a constant or a variable region of said α and/or β chain.

A TCR of the present invention may comprise only one of the α-chain or β-chain sequences as defined herein (in combination with a further α-chain or β-chain, respectively) or may comprise both chains.

In the context of the present invention, a "functional" T-cell receptor (TCR) α- and/or β-chain fusion protein shall mean an α- and/or β-chain fusion protein that, although the chain includes a (foreign) amino acid sequence or peptide, maintains at least substantial biological activity in the fusion protein. In the case of the α- and/or β-chain of a TCR, this shall mean that both chains remain able to form a T-cell receptor (either with a non-modified α- and/or β-chain or with another fusion protein α- and/or β-chain) which exerts its biological function, in particular binding to the specific peptide-MHC complex of said TCR, and/or functional signal transduction upon peptide activation.

As outlined above, the present functional TCR fusion protein has two basic functions: one is directed against an MHC-presented epitope, the other is a peptide-based or peptide-guided function in order to target an antigen. The peptide-guided function can in principle be achieved by introducing peptide sequences into the TCR and by targeting tumors with these peptide sequences. These peptides may be derived from phage display or synthetic peptide library (see supra, Arap et al. 1998; Scott 2001). Among others, peptides specific for breast, prostate and colon carcinomas as well as those specific for neo-vasculatures were already successfully isolated and may be used in the present invention (Samoylova et al. 2006).

In a preferred embodiment, the epitope is a tumor cell epitope and the antigen is a tumor cell antigen. Such a tumor cell epitope may be derived from a wide variety of tumor antigens such as antigens from tumors resulting from mutations, shared tumor specific antigens, differentiation antigens, and antigens overexpressed in tumors. Those antigens, for example may be derived from alpha-actinin-4, ARTC1, BCR-ABL fusion protein (b3a2), B-RAF, CASP-5, CASP-8, beta-catenin, Cdc27, CDK4, CDKN2A, COA-1, dek-can fusion protein, EFTUD2, Elongation factor 2, ETV6-AML1 fusion protein, FLT3-ITD, FN1, GPNMB, LDLR-fucosyltransferaseAS fusion protein, HLA-A2^{d}, HLA-A11^{d}, hsp70-2, KIAAO205, MART2, ME1, MUM-1^{f}, MUM-2, MUM-3, neo-PAP, Myosin class I, NFYC, OGT, OS-9, p53, pml-RARalpha fusion protein, PRDX5, PTPRK, K-ras, N-ras, RBAF600, SIRT2, SNRPD1, SYT-SSX1- or -SSX2 fusion protein, TGF-betaRII, triosephosphate isomerase, BAGE-1, GAGE-1, 2, 8, Gage 3, 4, 5, 6, 7, GnTV^{f}, HERV-K-MEL, KK-LC-1, KM-HN-1, LAGE-1, MAGE-A1, MAGE-A2, MAGE-A3, MAGE-A4, MAGE-A6, MAGE-A9, MAGE-A10, MAGE-A12, MAGE-C2, mucin^{k}, NA-88, NY-ESO-1/LAGE-2, SAGE, Sp17, SSX-2, SSX-4, TAG-1, TAG-2, TRAG-3, TRP2-INT2^{g}, XAGE-1b, CEA, gp100/Pmel17, Kallikrein 4, mammaglobin-A, Melan-A/MART-1, NY-BR-1, OA1, PSA, RAB38/NY-MEL-1, TRP-1/gp75, TRP-2, tyrosinase, adipophilin, AIM-2, ALDH1A1, BCLX (L), BING-4, CPSF, cyclin D1, DKK1, ENAH (hMena), EP-CAM, EphA3, EZH2, FGF5, G250/MN/CAIX, HER-2/neu, IL13Ralpha2, intestinal carboxyl esterase, alpha fetoprotein, M-CSFT, MCSP, mdm-2, MMP-2, MUC1, p53, PBF, PRAME, PSMA, RAGE-1, RGS5, RNF43, RU2AS, secernin 1, SOX10, STEAP1, surviving, Telomerase, VEGF, or WT1, just to name a few.

Further information in this regard may be derived from the data described in Nucleic Acids Research, 2006, Vol. 34, Database issue D607-D612; in tables 3a) and b) of Folkman et al., Angiogenesis: an organizing principle for drug discovery?, Nature Reviews Drug Discovery, Vol. 6, April 2007; and in table 1 of Langenkamp et al., Microvascular endothelial cell heterogeneity: general concepts and pharmacological consequences for anti-angiogenic therapy of cancer, Cell Tissue Res (2009) 335:205-222.

In a further embodiment, peptide sequences introduced into the TCR are providing a peptide-guided recognition and binding of an antigen which is a cell-surface antigen, or an extracellular antigen, preferably a neo-vasculature specific antigen.

The TFP of the present invention shows 3 different functionalties: the first is the function of the TCR α and/or β chain fusion protein to recognize and bind to at least one MHC-presented epitope; the second and third are based on amino acid sequences recognizing and binding an antigen, for example a cell-surface antigen and/or an extracellular antigen. It has never been shown before that a TFP having 3 functionalities can be expressed and will maintain all of these 3 functionalities without interfering with the TCR function as such.

It is referred to Fig. 2 and Fig. 8 providing examples of such a trifunctional TFP. As it can be seen from Fig. 9, it could be shown that these TFP's may be effectively expressed on cells (here Jurkat MA cells).

A preferred example of such a trifunctional TFP is one recognizing and binding to an MHC-presented tumor cell epitope; recognizing and binding a cell-surface antigen of a tumor cell and an extracellular tumor antigen, in particular a neo-vasculature specific antigen. This effectively provides an additional MHC independent activity of the TFP leading to the apoptosis of tumor cells and to an inhibition of angiogenesis at a time and thus helps to avoid the appearance of tumor escape mechanisms.

The cell surface antigen or the further MHC-presented epitope preferably is derived from HER2, CEA, PSMA, CD20 or one or more of the above mentioned antigens. The epitope is preferably selected from gp100 or gp1, or as well from one or more of the above mentioned antigens.

In a preferred embodiment, the amino acids (or peptides) recognizing and binding to an antigen are targeting a neo-vasculature specific antigen. Those antigens are valuable and promising targets in the attack on tumor cells. See, in this connection the above mentioned publications of Folkman et al., Angiogenesis: an organizing principle for drug discovery?, Nature Reviews Drug Discovery, Vol. 6, April 2007, and Langenkamp et al., Microvascular endothelial cell heterogeneity: general concepts and pharmacological consequences for anti-angiogenic therapy of cancer, Cell Tissue Res (2009) 335:205-222.

Preferably, the neo-vasculature specific antigen is integrin αvβ3 or αvβ5. For a further selection of antigens, see also table 3a) and b) of Folkman et al. and table 1 of Langenkamp et al.

According to the invention, the TCR fusion protein of the invention is characterized in that
i) the one or more antigen binding amino acid (peptide) sequences are added to the N-terminus of the α and/or β chain,
ii) the one or more antigen binding amino acid sequences are inserted into a constant or a variable region of said α and/or β chain, and/or
iii) the one or more antigen binding amino acid sequences are replacing a number of amino acids in a constant or a variable region of said α and/or β chain.

This selection of insertion sites of the amino acid sequence guarantees a proper function of the overall TCR, i.e. its biological function, in particular binding to the specific peptide-MHC complex of said TCR, and/or functional signal transduction upon peptide activation.

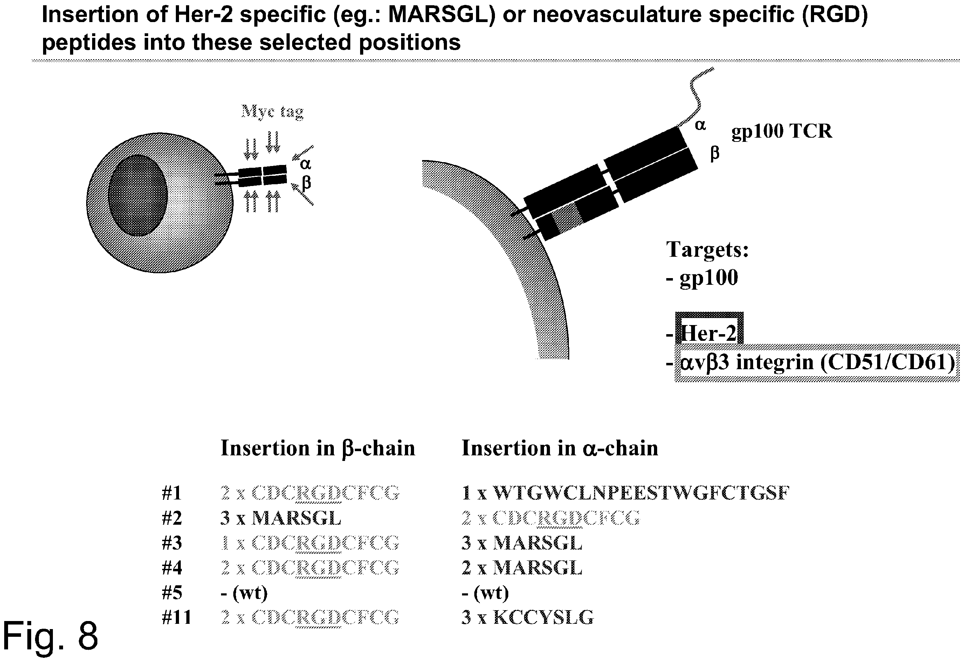

In a preferred embodiment, the one or more antigen binding amino acid (peptide) sequences are added to the N- terminus of the α and/or β chain, and/or are replacing a number of amino acids in the constant region of the β chain. More precisely, it is more preferred to replace the loop region of the constant region of the β chain with the respective antigen binding amino acid sequence. It is referred to Fig. 8 showing examples of this approach: clones #1, 2, 3, 4 and 11 each carry an added antigen binding amino acid sequence at the N-terminus of the α chain and one further antigen binding amino acid sequence replacing parts of the loop region of the constant region of the β chain. This combination of one addition at the N terminus of the α chain and one replaced sequence in the β chain constant region (replacing parts of the β loop) is the most preferred embodiment of an insertion scheme for the trifunctional TFP of the present invention. The replaced region may be located at aa positions 242 to 255 of the β chain constant region, for example 244-253, 246 to 255, 244 to 253 or 242 to 251.

An alternative, but less preferred location for inserting antigen binding amino acid sequences is the α chain constant region, for example at aa positions 170-179 (there is no loop region present in the α chain constant region).

Generally, replacement of sequences in the constant regions is preferred, but an insertion is also possible.

It is noted that the TFP of the present invention, in contrast to those of the prior art, does not require any peptide linker in order to link the individual components.

Preferably, said fusion protein comprises the one or more antigen binding amino acid sequences either spaced apart or directly in tandem, or the TCR is expressed as a single chain or soluble receptor. More preferred, the TFP of the present invention is expressed membrane-bound in order to achieve its full functionality.

In a further embodiment, the TCR α and/or β chain fusion protein of the invention may further comprise at least one epitope-tag, wherein said epitope-tag is selected from
i) an epitope-tag added to the N-terminus of the α and/or β chain,
ii) an epitope-tag inserted into a constant or variable region of said α and/or β chain, and iii) an epitope-tag replacing a number of amino acids in a constant or variable region of said α and/or β chain.

Preferred is a functional T-cell receptor (TCR) α- and/or β-chain fusion protein according to the present invention, wherein said epitope-tag is selected from, but not limited to, CD20 or HER2/neu tags, or other conventional tags such as a myc-tag, FLAG-tag, T7-tag, HA (hemagglutinin)-tag, His-tag, S-tag, GST-tag, or GFP-tag. myc, T7, GST, GFP tags are epitopes derived from existing molecules. In contrast, FLAG is a synthetic epitope tag designed for high antigenicity (see, e.g., U.S. Pat. Nos. 4,703,004 and 4,851,341). The myc- tag can preferably be used because high quality reagents are available to be used for its detection. Epitope tags can of course have one or more additional functions, beyond recognition by an antibody. The sequences of these tags are described in the literature and well known to the person of skill in art.

In the functional T-cell receptor (TCR) α- and/or β-chain fusion protein according to the present invention, said fusion protein may be for example selected from two myc-tag sequences that are attached to the N-terminus of an α-TCR-chain and/or 10 amino acids of a protruding loop region in the β-chain constant domain being exchanged for the sequence of two myc-tags.

In an embodiment of the present invention, the inventors inserted an amino acid sequence that corresponds to a part of the myc protein (myc-tag) at several reasonable sites into the structure of a T cell receptor and transduced this modified receptor into T cells. By introducing a tag into the TCR structure, it is possible to deplete the modified cells by administering the tag-specific antibody to the patient.

Those functional TCR fusion proteins may be used in a method for selecting a host cell population expressing a fusion protein selected from the group consisting of a fusion protein comprising a) at least one epitope-providing amino acid sequence (epitope-tag), and b) the amino acid sequence of an α- and/or β-chain of a TCR as defined above, wherein said epitope-tag is selected from an epitope-tag added to the N-terminus of said α- and/or β-chain or added into the α- and/or β-chain sequence, but outside the CDR3 region, an epitope-tag inserted into a constant region of said α- and/or β-chain, and an epitope-tag replacing a number of amino acids in a constant region of said α- and/or β-chain; and a TCR comprising at least one fusion protein as above on the surface of the host cell; comprising contacting host cells in a sample with a binding agent that immunologically binds to the epitope-tag, and selection of said host cells based on said binding.

In the TCR fusion protein, the amino acid sequence recognizing and binding an antigen has preferably a length of between 5-20 amino acids, more preferably between 6 and 12 amino acids. The amino acid sequence recognizing and binding an antigen is preferably selected from SEQ ID NO: 1 and/or 2 corresponding to MARSGL and CDCRGDCFC.

In a second aspect, the present invention provides an isolated nucleic acid coding for the TCR fusion protein as defined herein.

The term "isolated nucleic acid" as used herein with reference to nucleic acids refers to a naturally-occurring nucleic acid that is not immediately contiguous with both of the sequences with which it is immediately contiguous (one on the 5' end and one on the 3' end) in the naturally-occurring genome of the cell from which it is derived. For example, a nucleic acid can be, without limitation, a recombinant DNA molecule of any length, provided one of the nucleic acid sequences normally found immediately flanking that recombinant DNA molecule in a naturally-occurring genome is removed or absent. Thus, a nucleic acid includes, without limitation, a recombinant DNA that exists as a separate molecule (e. g., a cDNA or a genomic DNA fragment produced by PCR or restriction endonuclease treatment) independent of other sequences as well as recombinant DNA that is incorporated into a vector, an autonomously replicating plasmid, a virus (e. g., a retrovirus, or adenovirus). In addition, an isolated nucleic acid can include a recombinant DNA molecule that is part of a hybrid or fusion nucleic acid sequence.

Furthermore, the term "isolated nucleic acid" as used herein also includes artificially produced DNA or RNA sequences, such as those sequences generated by DNA synthesis based on *in silico* information.

The nucleic acids of the invention can comprise natural nucleotides, modified nucleotides, analogues of nucleotides, or mixtures of the foregoing as long as they are capable of causing the expression of a polypeptide *in vitro,* and preferably, in a T cell. The nucleic acids of the invention are preferably RNA, and more preferably DNA, but may also take the form of PNA, CNA, mRNA or mixtures thereof.

In a third aspect, the invention is directed to a vector, preferably a plasmid, shuttle vector, phagemide, cosmid, expression vector, retroviral vector, adenoviral vector or particle and/or vector to be used in gene therapy, which comprises the above defined nucleic acid molecule.

In the context of the present invention, a "vector" shall mean a nucleic acid molecule as introduced into a host cell, thereby producing a transformed host cell. A vector may include nucleic acid sequences that permit it to replicate in a host cell, such as an origin of replication. A vector may also include one or more selectable marker genes and other genetic elements known to those of ordinary skill in the art. A vector preferably is an expression vector that includes a nucleic acid according to the present invention operably linked to sequences allowing for the expression of said nucleic acid.

A fourth aspect provides a host cell transfected with a vector or a nucleic acid as defined above , or which has been infected or transduced with a particle as described above.

The host cell preferably is a T-cell, a natural killer cell, a monocyte, a natural killer T-cell, a monocyte- precursor cell, a hematopoietic stem cell or a non-pluripotent stem cell. More preferably, it is a peripheral blood lymphocyte (PBL) which has been transfected or transduced with the above vector. The step of cloning the T cell receptor (TCR) of the isolated T cells and/or expressing the TCR transgenes in PBMC can be done according to established methods.

In a still further preferred embodiment, the host cell expresses a fusion protein as disclosed herein on its surface.

A fifth aspect of the present invention provides a method for producing a fusion protein according to the invention, comprising a chemical synthesis or genetic engineering of said peptide. Those methods for chemically synthesizing proteins and peptides are described in the literature, and well known to the person of skill.

Peptides (at least those containing peptide linkages between amino acid residues) may be synthesized by the Fmoc-polyamide mode of solid-phase peptide synthesis as disclosed by Lu et al (1981) J. Org. Chem. 46, 3433-3436, and references therein. Temporary N-amino group protection is afforded by the 9-fluorenylmethyloxycarbonyl (Fmoc) group. Repetitive cleavage of this highly base-labile protecting group is achieved by using 20 % piperidine in N, N-dimethylformamide. Side-chain functionalities may be protected as their butyl ethers (in the case of serine threonine and tyrosine), butyl esters (in the case of glutamic acid and aspartic acid), butyloxycarbonyl derivative (in the case of lysine and histidine), trityl derivative (in the case of cysteine) and 4-methoxy-2,3,6-trimethylbenzenesulphonyl derivative (in the case of arginine). Where glutamine or asparagine are C-terminal residues, use is made of the 4,4'-dimethoxybenzhydryl group for protection of the side chain amido functionalities. The solid-phase support is based on a polydimethyl-acrylamide polymer constituted from the three monomers dimethylacrylamide (backbone-monomer), bisacryloylethylene diamine (cross linker) and acryloylsarcosine methyl ester (functionalizing agent). The peptide-to-resin cleavable linked agent used is the acid-labile 4-hydroxymethyl- phenoxyacetic acid derivative. All amino acid derivatives are added as their preformed symmetrical anhydride derivatives with the exception of asparagine and glutamine, which are added using a reversed N, N-dicyclohexyl-carbodiimide/lhydroxybenzotriazole mediated coupling procedure. All coupling and deprotection reactions are monitored using ninhydrin, trinitrobenzene sulphonic acid or isotin test procedures.

Upon completion of synthesis, peptides are cleaved from the resin support with concomitant removal of side-chain protecting groups by treatment with 95% trifluoroacetic acid containing a 50 % scavenger mix. Scavengers commonly used are ethanedithiol, phenol, anisole and water, the exact choice depending on the constituent amino acids of the peptide being synthesized. Trifluoroacetic acid is removed by evaporation in vacuo, with subsequent trituration with diethyl ether affording the crude peptide. Any scavengers present are removed by a simple extraction procedure which on lyophilization of the aqueous phase affords the crude peptide free of scavengers. Reagents for peptide synthesis are generally available from Calbiochem- Novabiochem (UK) Ltd, Nottingham NG7 2QJ, UK.

Purification may be effected by any one, or a combination of, techniques such as size exclusion chromatography, ion-exchange chromatography and (usually) reverse-phase high performance liquid chromatography.

Analysis of peptides may be carried out using thin layer chromatography, reverse-phase high performance liquid chromatography, amino-acid analysis after acid hydrolysis and by fast atom bombardment (FAB) mass spectrometric analysis, as well as MALDI and ESI-Q-TOF mass spectrometric analysis.

Furthermore, the fusion protein may be prepared by using genetic engineering: such a method, as an example, may comprise expressing a nucleic acid molecule encoding a fusion protein according to the present invention in a host cell of the invention, and purifying said fusion protein or said TCR from said host cell.

One skilled in the art will understand that there are myriad ways to express a recombinant protein such that it can subsequently be purified. In general, an expression vector carrying the nucleic acid sequence that encodes the desired fusion protein will be transformed into a microorganism for expression. Such microorganisms can be prokaryotic or eukaryotic. A eukaryotic expression system will be preferred where the protein of interest requires eukaryote-specific post-translational modifications such as glycosylation. Also, protein can be expressed using a viral (e.g., vaccinia) based expression system.

Protein can also be expressed in animal cell tissue culture, and such a system will be appropriate where animal-specific protein modifications are desirable or required in the recombinant protein. Such expression is particularly appropriate where native assembly and export of the inventive fusion protein is desirable, since the activity of TCRs is influenced by native dimerization (folding and assembly) and presentation on the cell.

In a sixth aspect, the present invention provides a pharmaceutical composition which comprises a fusion protein, a nucleic acid molecule, a vector according or a host cell as defined hereinabove, together with a pharmaceutically acceptable carrier or excipient.

Those active components of the present invention are preferably used in such a pharmaceutical composition, in doses mixed with an acceptable carrier or carrier material, that the disease can be treated or at least alleviated. Such a composition can (in addition to the active component and the carrier) include filling material, salts, buffer, stabilizers, solubilizers and other materials, which are known state of the art.

The term "pharmaceutically acceptable" defines a non-toxic material, which does not interfere with effectiveness of the biological activity of the active component. The choice of the carrier is dependent on the application.

The pharmaceutical composition can contain additional components which enhance the activity of the active component or which supplement the treatment. Such additional components and/or factors can be part of the pharmaceutical composition to achieve synergistic effects or to minimize adverse or unwanted effects.

Techniques for the formulation or preparation and application/medication of active components of the present invention are published in "Remington's Pharmaceutical Sciences", Mack Publishing Co., Easton, PA, latest edition. An appropriate application is a parenteral application, for example intramuscular, subcutaneous, intramedular injections as well as intrathecal, direct intraventricular, intravenous, intranodal, intraperitoneal or intratumoral injections. The intravenous injection is the preferred treatment of a patient. According to a preferred embodiment, the pharmaceutical composition is an infusion or an injection.

An injectable composition is a pharmaceutically acceptable fluid composition comprising at least one active ingredient, e.g., an expanded T-cell population (for example autologous or allogenic to the patient to be treated) expressing a TCR. The active ingredient is usually dissolved or suspended in a physiologically acceptable carrier, and the composition can additionally comprise minor amounts of one or more non-toxic auxiliary substances, such as emulsifying agents, preservatives, and pH buffering agents and the like. Such injectable compositions that are useful for use with the fusion proteins of this disclosure are conventional; appropriate formulations are well known to those of ordinary skill in the art.

According to a further aspect, the present specification is directed to a method of treating a patient in need of adoptive cell therapy, said method comprising administering to said patient a pharmaceutical composition as defined above. The disease to be treated preferably is a tumor, autoimmune, hereditary, or infectious disease, immunodeficiency, allergy and/or the composition will be designed for use in transplantation.

The present invention now will be illustrated by the enclosed Figures and the Examples. The following examples further illustrate the invention but, of course, should not be construed as limiting its scope.

### DESCRIPTION OF THE FIGURES

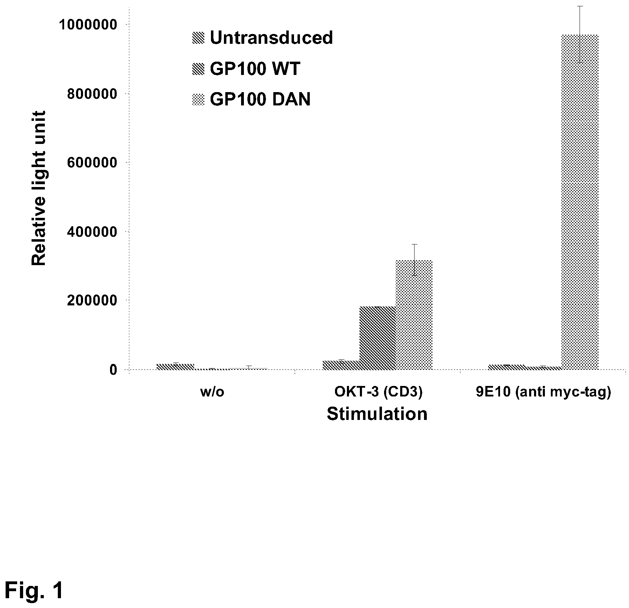

Figure 1: Jurkat MA T cells untransduced or transduced with the wilde-type (WT) or with the myc-taged gp 100 (DAN) TCRs were unstimulated or stimulated with a CD3 (OKT-3) antibody as a positive control, or with an anti myc-tag (9E10) antibody. Response was measured using the luciferin conversion assay and measured in relative light unit.
Figure 2: Schematic representation of the TCR (left) and the TFPhv (right) with the HER-2 specific peptide (MARSGL) and the neo-vasculature integrins αvβ3 and αvβ5 specific peptide (RGD4C) are depicted at the N-termini of the TCR molecule.

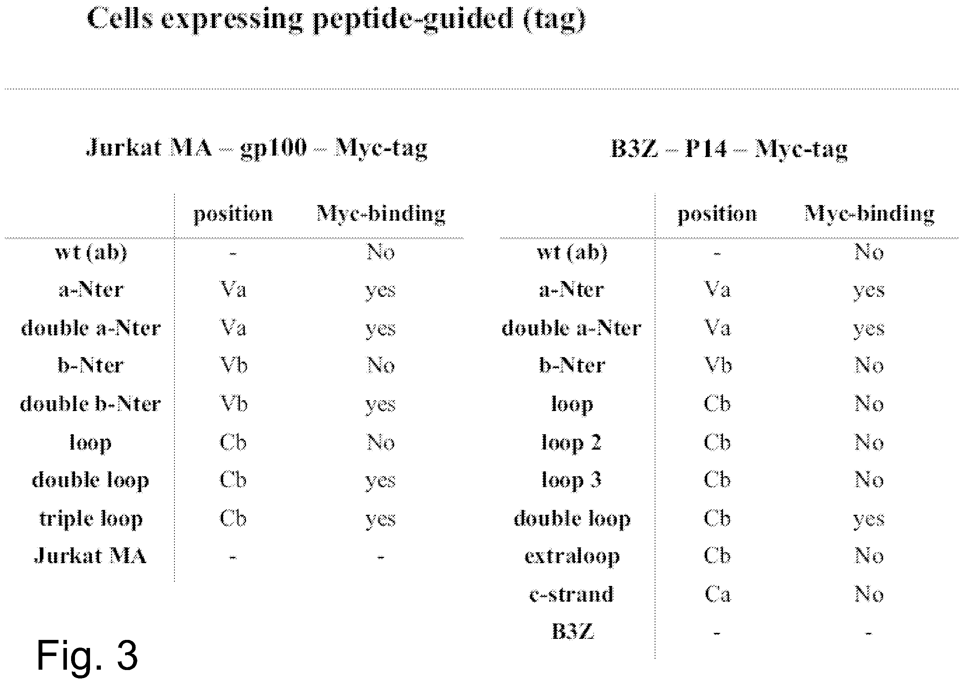

Figure 3: Overview over two cell types (Jurkat MA and B3Z) expressing peptide-guided TCR's (bifunctional). The insertion positions and myc-binding are indicated in the tables.

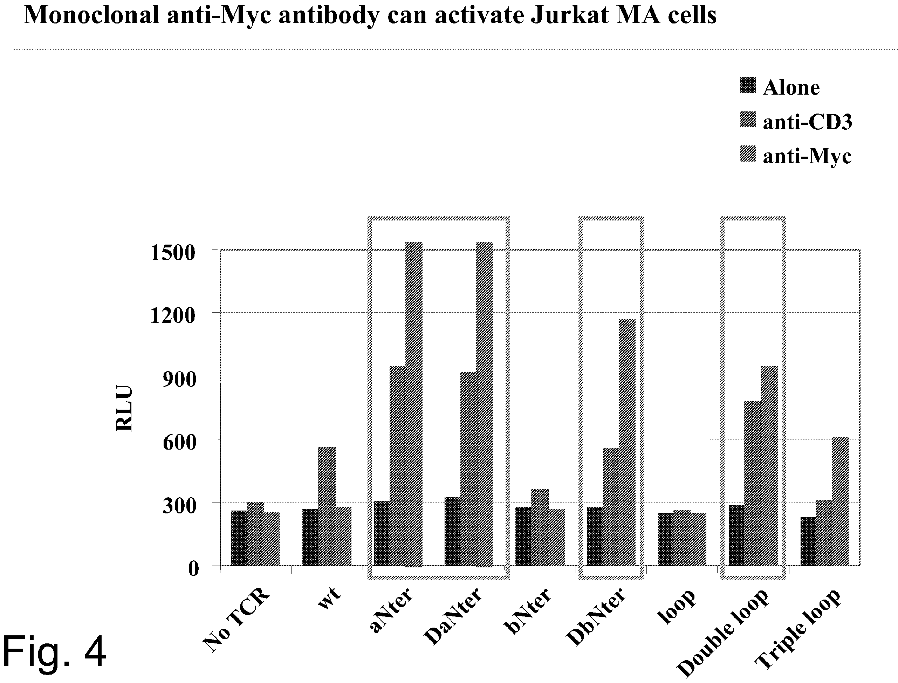

Figure 4: Monoclonal anti-Myc antibody can activate Jurkat MA cells. Jurkat MA T cells were activated with anti-CD3 or anti-Myc-tag antibody. The activation was measured in relative light unit (RLU), indicating IL-2 secretion, and compared to wild type (wt).

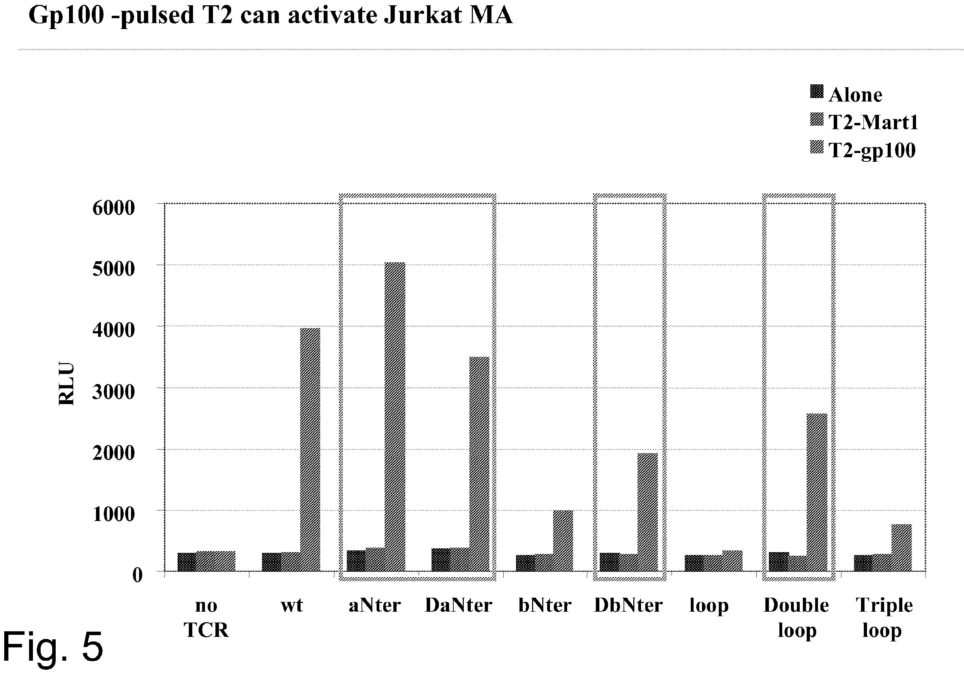

Figure 5: Gp100 -pulsed T2 can activate Jurkat MA. Jurkat MA T cells were cultivated with T2 cells loaded with the peptide specific for the TCR (gp100) or with an irrelevant peptide (Mart1). The activation were measured in relative light unit (RLU), indicating IL-2 secretion, and compared to wild type (wt).

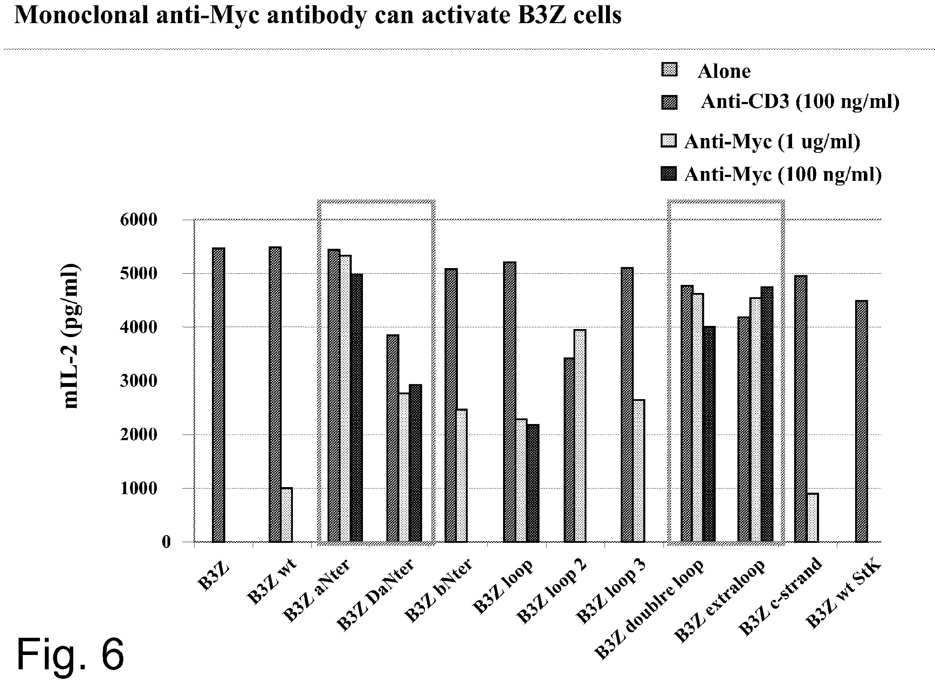

Figure 6: Monoclonal anti-Myc antibody can activate B3Z cells. B3Z cells were activated with anti-CD3 or anti-Myc-tag antibody. The activation were measured by IL-2 secretion, and compared to wild type (wt).

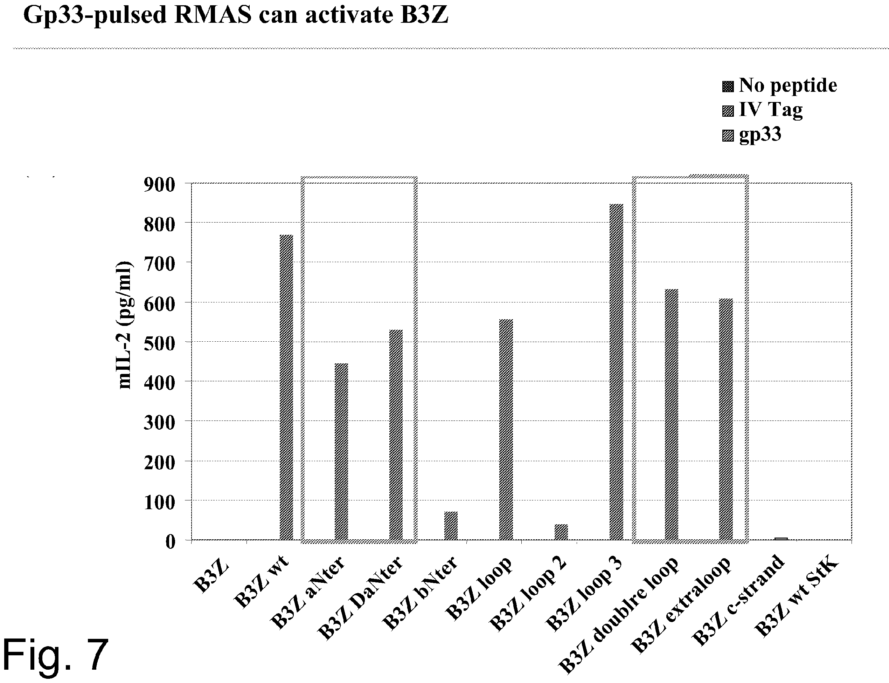

Figure 7: Gp33-pulsed RMAS can activate B3Z. B3Z cells were cultivated with RMAS cells loaded with the peptide specific for the TCR (gp33) or with an irrelevant peptide (IV Tag). The activation were measured by IL-2 secretion, and compared to wild type (wt).
Figure 8: Insertion of Her-2 specific (eg.: MARSGL) or neovasculature specific (RGD) peptides into these selected positions.

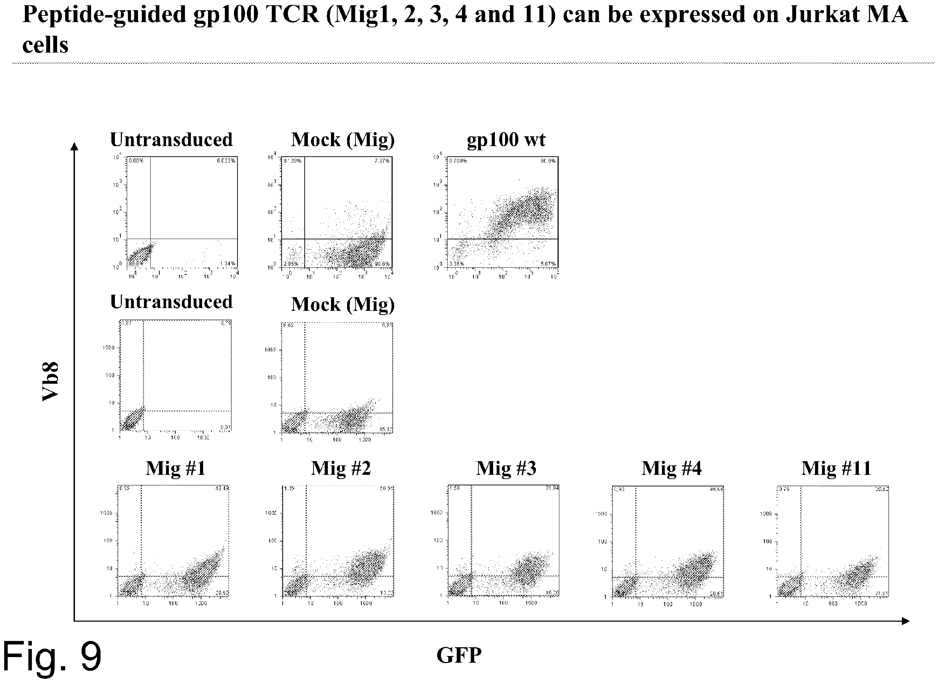

Figure 9: Peptide-guided gp100 TCR (Mig 1, 2, 3, 4 and 11) can be expressed on Jurkat MA cells.

### EXAMPLES

The P14 TCR, which recognises the gp33 epitope from the lymphocytic choriomeningitis virus, was studied for identifying neutral sites for the introduction of peptide sequences. Similarly, the gp100 TCR, which recognises the human melanoma antigen derived epitope gp100₂₀₉ was studied for identifying such sites. This was achieved by introducing a myc-tag sequence (table 1) into 11 different sites of the TCR molecule, five of which were used in both TCRs resulting in a total number of 16 different TFPs. T cells transduced with any of these 16 constructs expressed the TFP at a level similar to that of the wild type P14 or gp100 TCRs and maintained their specificity to the corresponding tetramer and to stimulation by the specific peptide-pulsed target.

### Testing TFP for the ability of responding to de novo stimulation:

The inventors have recently used CD3 or myc-tag specific antibodies to investigate the possibility of stimulating the transduced T cells. They found that 9 of the 16 TFPs located in 3 different positions of the TFPs can be triggered by the myc-tag specific antibody to produce similar amount of IL-2 to that produced through the control stimulation by the CD3 antibody (Fig. 1). These data indicate that a targeting-peptide introduced to the TCR can be used to trigger the TCR similarly to the triggering of this molecule by the corresponding MHC-peptide complex and provides 3 potential sites for the introduction of targeting peptides.

### Molecular cloning of TFP encompassing TCR with three specificities:

Peptide sequences specific for the tumor-associated antigen HER-2 and/or tumor vasculature (based on the RGD4C sequence) indicated in table 1 are cloned into the identified optimal sites of the TCR sequences. This is achieved by introducing the corresponding nucleotide sequences into the TCR chains using overlapping PCR primers.

Unique restriction sites with silent substitutions are introduced to enable a potential further cloning of alternative targeting peptides. Constructs identities are confirmed by restriction mapping and sequencing. The resulting TFPs recognise HER-2, neovasculature and gp33-expressing H2Kb target cells or gp100₂₀₉ HLA-A2-expressing target cells. Three different types of TFPs with specificities to TCR epitope and HER-2 (TFPh), TCR epitope and angiogenesis (TFPv) or TCR epitope, HER-2 and angiogenesis (TFPhv) are constructed (Fig.2).

### Establishing a tumor cell line with two different model antigens with the expression level of one being controllable:

A tumor cell line is constructed in which the level of HER-2 will be constant, while the level of gp33 is controlled by an inducible cre recombination system based on the MC57G-gp33 cell line provided by Dr. H. Schreiber, Chicago. In this system the gp33 eptitope is expressed at low but detectable levels due to inhibition by a floxable sequence containing the HLA-A2 and puromycin. Following cre recombination, which is induced by tamoxifen administration, this inhibitory sequence is floxed, which enables a higher expression level for the gp33 epitope. The uninduced tumor cell line is designated MC57G-gp33_{low}, and the induced cell line is designated MC57G-gp33_{high}. In this model a control cell line MC57G-neo, which expresses the inducible cre construct is used as a gp33 negative cell line.

HER-2 is introduced to the uninduced MC57G-gp33 or to MC57G-neo by retroviral transduction using the MIG-R1 HER-2 construct (provided by Dr. Helga Bernhard, GSF, Munich). The resulting tumor cell lines are designated MC57G-neo-HER-2, MC57G-gp33_{low}-HER-2, and MC57G-gp33_{high}-HER-2.

### T cell transduction

Human T cells are derived from peripheral blood lymphocytes, while mouse T cells are derived from splenocytes of OT-I-Rag-/- TCR transgenic mice.

T cells are transduced with the different TFPs by spinocculation. Cryopreserved peripheral blood mononuclear cells are collected from healthy donor blood donation by centrifugation on Ficoll-Hypaque gradients. Peripheral blood mononuclear cells are stimulated with 10 ng/mL CD3 (ORTHOCLONE OKT3) and 600 IU IL-2/mL. The retroviral production is done in 293T cells that are cotransfected with the TFP encoding plasmid or GFP as a mock control and the pCL-10A1 plasmid encoding gag, pol and env using lipofection. Virus supernatant is collected and used to transduce primary activated human T cells three days after the activation. Enrichment of mouse T cells for retroviral transduction is achieved by isolating splenocytes from OT-I mice, followed by red blood cell lyses by ammonium chloride solution and stimulation with the OT-I epitope (SIINFEKL), anti CD3 and anti CD28 antibodies. The ecotropic virus supernatant is produced by transiently transfecting plasmid-DNA encoding the appropriate TFP or GFP as a mock control using lipofection into the ecotropic packaging cell line Plat-E. T cells are transduced on two subsequent days and the transduction efficacy will be analysed by FACS.

### Measuring TFP expression and binding capacity to three independent antigens

TFPs expression is confirmed using specific antibodies, tetramers and by using recombinant proteins followed by specific antibody staining. This is achieved using commercially available recombinant HER-2 or αvβ3 (which binds to the RGD4C peptide) proteins and antibodies specific to these proteins. Cells expressing the TFPs or control TCRs are incubated with the recombinant protein for 30 minutes after which the residual non-binding protein is washed away. Fluorochrome-conjugated antibodies specific for either of the recombinant proteins are then added to the cells and incubated for further 30 min followed by washing and fixing with 4% paraformaldehyde solution. Binding is then detected by FACS. Specificity is confirmed by adding a competing soluble synthetic peptide.

### In vitro analysis of the functional activity of TFP

TFPs function is confirmed using cytokine release assay with the transduced cells as effectors and target cell lines expressing any or all of the target antigens. HUVECS is used as target for the vasculature targeting as well as tumor cell lines expressing αvβ3 such as MDAMB435 breast carcinoma. To evaluate HER-2 specificity of the TFP, C1R/A2 and its transfectant C1R/A2HER2, which expresses HER-2 are used as well as carcinoma cell lines expressing HER-2. Functional specificity is confirmed by adding a competing soluble synthetic peptide. TFPs specificity for the gp33 and gp100₂₀₉ epitopes are confirmed by using peptide-pulsed RMA-S and T2 cells, respectively, and by using MC57-neo and MC57-gp33 expressing tumor cell lines and 624-28 (HLA-A2 negative and gp100 positive) and 624-38 (HLA-A2 and gp100 positive) melanoma cell lines.

### Adoptive therapy with TFP-engineered T cells targeting antigen-loss variants

The rational for this study is to answer the question of whether it is possible to provide an immunotherapeutic strategy that prevents "immune escape" of tumor cells expressing low amount of a targeted antigen. An MHC-restricted epitope, a cell surface-expressed proto-oncogene and tumor vasculature are targeted simultaneously.

MC57G tumor cell lines are used to compare the therapeutic potential of P14 TCR versus the TFPs based on P14 with the added specificity to HER-2 and neo-vasculature. Six different populations of T cells are used in the adoptive transfer:
1. OT-I
2. P14 T cells from P14 TCR transgenic mice
3. P14 transduced OT-I
4. P14 TFP transduced OT-I with specificities to H2Kb-gp33 and HER-2 (TFPh)
5. P14 TFP transduced OT-I with specificities to H2Kb-gp33 and angiogenesis (TFPv)
6. P14 TFP transduced OT-I with specificities to H2Kb-gp33, HER-2 and angiogenesis (TFPhv)

Rag-/- OT-I mice are challenged subcutaneously on contra-lateral sites with either of the following 4 tumor pairs:
A. MC57-neo and MC57-gp33_{low}
B. MC57-neo and MC57G-neo-HER-2
C. MC57G-neo-HER-2 and MC57G-gp33_{low}-HER-2
D. MC57G-neo and MC57G-gp33_{high}

Once the tumors reach 1 cm mean tumor diameter, each of the 4 groups receive intravenous injection with 10e6 activated CD8+ positive P14, OT-I, P14 transduced OT-I, TFP transduced OT-I T cells or TFP transduced OT-I T cells and the RGD4C synthetic peptide, which are used to block the recognition of the RGD4C sequence of the TFP. Efficient tumor destruction would require targeting tumor stroma either directly through RGD4C specificity of the TFP or through targeting stroma cells cross presenting the gp33 epitope. The expected recognition spectrum of these different effectors is outlined in table 2.

**Table 1. Amino acid sequences of peptides used in the TFPs.**

| **Peptide** | **Target** | **Sequence** | **Length aa** |
|---|---|---|---|
| myc-tag | myc antibody | EQKLISEEDL (SEQ ID NO:3) | 10 |
| 2xmyc-tag | myc antibody | EQKLISEEDLEQKLISEEDL (SEQ ID NO:4) | 20 |
| MARS | HER-2 | MARSGL (SEQ ID NO:1) | 6 |
| RGD4C | αv Integrins | CDCRGDCFC (SEQ ID NO:2) | 9 |

**Table 2. The predicted recognition pattern of transduced/transferred T cells.**

| | **Transferred T cells and recognition** | | | | | | |
|---|---|---|---|---|---|---|---|
| **Target** | OT-I | P14 | P14-OT-I | TFPh-OT-I | TFPv-OT-I | TFPhv-OT-I | TFPhv-OT-I +RGD4C peptide |
| H2Kb-gp33 | No | Yes | Yes | Yes | Yes | Yes | Yes |
| HER-2 | No | No | No | Yes | No | Yes | Yes |
| Neo-vasculature | No | No | No | No | Yes | Yes | No |

### Publications:

Arap, W., R. Pasqualini and E. Ruoslahti (1998). "Cancer treatment by targeted drug delivery to tumor vasculature in a mouse model." Science 279(5349): 377-80.
Folkman, J. (2007). "Angiogenesis: an organizing principle for drug discovery?" Nat Rev Drug Discov 6(4): 273-86.
Leen, A. M., C. M. Rooney and A. E. Foster (2007). "Improving T cell therapy for cancer." Annu Rev Immunol 25: 243-65.
Samoylova, T. I., N. E. Morrison, L. P. Globa and N. R. Cox (2006). "Peptide phage display: opportunities for development of personalized anti-cancer strategies." Anticancer Agents Med Chem 6(1): 9-17.
Wang, X., H. Zhao, Q. Xu, W. Jin, C. Liu, H. Zhang, Z. Huang, X. Zhang, Y. Zhang, D. Xin, A. J. Simpson, L. J. Old, Y. Na, Y. Zhao and W. Chen (2006). "HPtaa database-potential target genes for clinical diagnosis and immunotherapy of human carcinoma." Nucleic Acids Res 34(Database issue): D607-12.
Becker, C., H. Pohla, B. Frankenberger, T. Schuler, M. Assenmacher, D. J. Schendel and T. Blankenstein (2001). "Adoptive tumor therapy with T lymphocytes enriched through an IFN-gamma capture assay." Nat Med 7(10): 1159-62.
Charo, J., S. E. Finkelstein, N. Grewal, N. P. Restifo, P. F. Robbins and S. A. Rosenberg (2005). "Bcl-2 overexpression enhances tumor-specific T-cell survival." Cancer Res 65(5): 2001-8.
Gladow, M., W. Uckert and T. Blankenstein (2004). "Dual T cell receptor T cells with two defined specificities mediate tumor suppression via both receptors." Eur J Immunol 34(7): 1882-91.
Kieback E, Charo J, Sommermeyer D, Blankenstein T, Uckert W (2008). A safeguard eliminates T cell receptor gene-modified autoreactive T cells after adoptive transfer. Proc Natl Acad Sci USA. 105(2):623-8.
Kruschinski A, Moosmann A, Poschke I, Norell H, Chmielewski M, Seliger B, Kiessling R, Blankenstein T, Abken H and J Charo (2008).Engineering antigen-specific primary human NK cells against HER-2 positive carcinomas. Proc Natl Acad Sci USA. 105(45): 17481-6.
Sommermeyer, D., J. Neudorfer, M. Weinhold, M. Leisegang, B. Engels, E. Noessner, M. H. Heemskerk, J. Charo, D. J. Schendel, T. Blankenstein, H. Bernhard and W. Uckert (2006). "Designer T cells by T cell receptor replacement." Eur J Immunol 36(11): 3052-9.

## Claims

1. A functional TCR α and/or β chain fusion protein recognizing and binding to at least one MHC-presented epitope, and containing at least two amino acid sequences recognizing and binding an antigen,
wherein the TCR α and/or β chain fusion protein comprises an α-chain or β-chain in combination with a further α-chain or β-chain, respectively, or comprises an α-chain and a β-chain,
wherein the epitope is a tumor cell epitope and the antigen is a tumor cell antigen, and wherein the TCR fusion protein is trifunctional, where the first function is the function of the TCR α and/or β chain fusion protein to recognize and bind to at least one MHC-presented epitope; the second and third are based on amino acid sequences recognizing and binding an antigen, and
wherein
i) the at least two antigen binding amino acid sequences are added to the N-terminus of the α and/or β chain,
ii) the at least two antigen binding amino acid sequences are inserted into a constant or a variable region of said α and/or β chain, and/or
iii) the at least two antigen binding amino acid sequences are replacing a number of amino acids in a constant or a variable region of said α and/or β chain.

2. The TCR fusion protein of claim 1, wherein the antigen is a cell-surface antigen or an extracellular antigen, preferably a neo-vasculature specific antigen.

3. The TCR fusion protein of claims 1 and 2, wherein the cell surface antigen is derived from HER2, CEA, or PSMA.

4. The TCR fusion protein of one or more of the preceding claims, wherein the epitope is selected from gp100 or gp1, or wherein the neo-vasculature specific antigen is integrin αvβ3 or αvβ5.

5. The TCR fusion protein of claim 1, wherein the at least two antigen binding amino acid sequences are added to the N-terminus of the α and/or β chain, and/or wherein the at least two antigen binding amino acid sequences are replacing a number of amino acids in the loop region of the β chain constant region.

6. The TCR fusion protein of one or more of the preceding claims, wherein said fusion protein comprises the at least two antigen binding amino acid sequences either spaced apart or directly in tandem, or wherein the TCR is expressed membrane-bound.

7. The TCR α and/or β chain fusion protein of one or more of the preceding claims, further comprising at least one epitope-tag, wherein said epitope-tag is selected from
i) an epitope-tag added to the N- and/or C-terminus of the α and/or β chain,
ii) an epitope-tag inserted into a constant or variable region of said α and/or β chain, and
iii) an epitope-tag replacing a number of amino acids in a constant or variable region of said α and/or β chain.

8. The TCR fusion protein of one or more of the preceding claims, wherein the amino acid sequence recognizing and binding an antigen has a length of between 5-20 amino acids, preferably between 6 and 12 amino acids, and wherein the amino acid sequence recognizing and binding an antigen preferably is selected from SEQ ID NO: 1 and/or 2.

9. An isolated nucleic acid molecule coding for the TCR fusion protein of one or more of the preceding claims.

10. The nucleic acid molecule according to claim 9, wherein said molecule is selected from DNA, RNA, PNA, CNA, mRNA or mixtures thereof.

11. A vector, preferably a plasmid, shuttle vector, phagemide, cosmid, expression vector, retroviral vector, adenoviral vector or particle and/or vector suitable for use in gene therapy, which comprises a nucleic acid molecule of claims 9 or 10.

12. A host cell, transfected with a vector of claim 11 or a nucleic acid of claim 9, or infected or transduced with a particle according to claim 11, wherein said cell preferably is a T-cell, a natural killer cell, a monocyte, a natural killer T-cell, precursor cell, a hematopoietic stem cell or a non-pluripotent stem cell, wherein said host cell expresses a fusion protein according to any of claims 1 to 9 on its surface.

13. A method for producing a fusion protein according to any of claims 1 to 8, comprising a chemical synthesis or genetic engineering of said peptide, or
comprising expressing a nucleic acid molecule in a host cell according to claim 12, and purifying said fusion protein or said TCR from said host cell.

14. A pharmaceutical composition, comprising a fusion protein according to any of claims 1 to 8, a nucleic acid molecule according to claim 9 or 10, a vector according to claim 11 or a host cell according to any of claims 12, together with a pharmaceutically acceptable carrier or excipient.

15. The pharmaceutical composition of claim 14 for use in the treatment of tumors, autoimmune, hereditary, or infectious diseases, immunodeficiency, allergy and/or for use in transplantation.

## Patentansprüche

1. Funktionelles TCR α und/oder β Ketten-Fusionsprotein, das mindestens ein MHC-präsentiertes Epitop erkennt und an dieses bindet und mindestens zwei Aminosäuresequenzen enthält, die ein Antigen erkennen und binden,
wobei das TCR α und/oder β Ketten-Fusionsprotein eine α-Kette oder β-Kette in Kombination mit jeweils einer weiteren α-Kette oder β-Kette umfasst oder eine α-Kette und eine β-Kette umfasst,
wobei das Epitop ein Tumorzellepitop ist und das Antigen ein Tumorzellantigen ist, und wobei das TCR-Fusionsprotein trifunktional ist, wobei die erste Funktion die Funktion des TCR α und/oder β Ketten-Fusionsproteins ist, wenigstens ein MHC-präsentiertes Epitop zu erkennen und an dieses zu binden; wobei die zweite und dritte auf Aminosäuresequenzen basiert, die ein Antigen erkennen und binden, und
worin
i) die mindestens zwei antigenbindenden Aminosäuresequenzen dem N-Terminus der α und/oder β Kette hinzugefügt werden,
ii) die mindestens zwei antigenbindenden Aminosäuresequenzen in eine konstante oder variable Region der α und/oder β Kette eingefügt werden, und/oder
iii) die mindestens zwei antigenbindenden Aminosäuresequenzen eine Anzahl von Aminosäuren in einer konstanten oder variablen Region der α und/oder β Kette ersetzen.

2. TCR-Fusionsprotein nach Anspruch 1, wobei das Antigen ein Zell-Oberflächen-Antigen oder ein extrazelluläres Antigen, vorzugsweise ein Neo-Vaskulatur spezifisches Antigen, ist.

3. TCR-Fusionsprotein nach Anspruch 1 und 2, wobei das Zelloberflächenantigen von HER2, CEA oder PSMA abgeleitet ist.

4. Das TCR-Fusionsprotein nach einem oder mehreren der vorgenannten Ansprüche, wobei das Epitop aus gp100 oder gp1 ausgewählt ist, oder wobei das Neo-Vaskulatur spezifische Antigen integrin-αvβ3 oder αvβ5 ist.

5. TCR-Fusionsprotein nach Anspruch 1, wobei die mindestens zwei antigenbindenden Aminosäuresequenzen dem N-Terminus der α und/oder β Kette hinzugefügt sind, und/oder wobei die mindestens zwei antigenbindenden Aminosäuresequenzen eine Anzahl von Aminosäuren in der Loop-Region der konstanten Region der β Kette ersetzen.

6. TCR-Fusionsprotein nach einem oder mehreren der vorstehenden Ansprüche, wobei das Fusionsprotein die mindestens zwei antigenbindenden Aminosäuresequenzen entweder voneinander beabstandet oder direkt im Tandem angeordnet umfasst, oder wobei das TCR membrangebunden exprimiert wird.

7. Das TCR α und/oder β Ketten-Fusionsprotein nach einem oder mehreren der vorstehenden Ansprüche, ferner umfassend mindestens ein Epitop-Tag, wobei das Epitop-Tag ausgewählt ist aus
i) einem Epitop-Tag, das dem N- und/oder C-Terminus der α und/oder β Kette hinzugefügt wird,
ii) einem Epitop-Tag, das in eine konstante oder variable Region der α und/oder β Kette eingefügt ist, und
iii) einem Epitop-Tag, der eine Anzahl von Aminosäuren in einer konstanten oder variablen Region der Kette α und/oder β ersetzt.

8. TCR-Fusionsprotein nach einem oder mehreren der vorstehenden Ansprüche, wobei die Aminosäuresequenz, die ein Antigen erkennt und bindet, eine Länge zwischen 5-20 Aminosäuren, vorzugsweise zwischen 6 und 12 Aminosäuren aufweist, und wobei die Aminosäuresequenz, die ein Antigen erkennt und bindet, vorzugsweise aus SEQ ID NO: 1 und/oder 2 ausgewählt ist.

9. Isoliertes Nukleinsäuremolekül, das für das TCR-Fusionsprotein eines oder mehrerer der vorhergehenden Ansprüche kodiert.

10. Nukleinsäuremolekül nach Anspruch 9, wobei das Molekül ausgewählt ist aus DNA, RNA, PNA, CNA, CNA, mRNA oder Mischungen davon.

11. Vektor, vorzugsweise Plasmid, Shuttle-Vektor, Phagemid, Cosmid, Expressionsvektor, retroviraler Vektor, adenoviraler Vektor oder Partikel und/oder Vektor, der zur Verwendung in der Gentherapie geeignet ist, umfassend ein Nukleinsäuremolekül nach Anspruch 9 oder 10.

12. Wirtszelle, transfiziert mit einem Vektor nach Anspruch 11 oder einer Nukleinsäure nach Anspruch 9, oder infiziert oder transduziert mit einem Partikel nach Anspruch 11, wobei die Zelle vorzugsweise eine T-Zelle, eine natürliche Killerzelle, ein Monozyt, eine natürliche Killer-T-Zelle, eine Vorläuferzelle, eine hämatopoetische Stammzelle oder eine nicht-pluripotente Stammzelle ist, wobei die Wirtszelle ein Fusionsprotein nach einem der der Ansprüche 1-9 auf ihrer Oberfläche exprimiert.

13. Verfahren zur Herstellung eines Fusionsproteins nach einem der Ansprüche 1 bis 8, umfassend eine chemische Synthese oder gentechnische Herstellung des Peptids, oder umfassend das Exprimieren eines Nukleinsäuremoleküls in einer Wirtszelle nach Anspruch 12 und das Aufreinigen des Fusionsproteins oder des TCR aus der Wirtszelle.

14. Pharmazeutische Zusammensetzung, umfassend ein Fusionsprotein nach einem der Ansprüche 1 bis 8, ein Nukleinsäuremolekül nach Anspruch 9 oder 10, einen Vektor nach Anspruch 11 oder eine Wirtszelle nach einem der Ansprüche 12, zusammen mit einem pharmazeutisch verträglichen Träger oder Hilfsstoff.

15. Pharmazeutische Zusammensetzung nach Anspruch 14 zur Verwendung bei der Behandlung von Tumoren, Autoimmun-, Erb- oder Infektionskrankheiten, Immunschwäche, Allergien und/oder zur Verwendung bei Transplantationen.

## Revendications

1. Protéine de fusion de chaîne α et/ou β d'un récepteur de lymphocyte T fonctionnel reconnaissant et se liant à au moins un épitope présenté du CMH (complexe majeur d'histocompatibilité), et conprenant au moins deux séquences d'acides aminés reconnaissantes et se liant à un antigène,
la protéine de fusion de chaîne α et/ou β du récepteur de lymphocyte T comprenant une chaîne α ou une chaîne β combinée avec une autre chaîne α ou une autre chaîne β, respectivement, ou comprenant une chaîne α et une chaîne β,
l'épitope étant un épitope de cellule tumorale et l'antigène étant un antigène de cellule tumorale, et
la protéine de fusion du récepteur de lymphocyte T étant trifonctionnelle, la première fonction étant la fonction de la protéine de fusion de chaîne α et/ou β du récepteur de lymphocyte T à reconnaître et à se lier à au moins un épitope présenté du CMH ; la seconde et la troisième fonction étant pour base les séquences d'acides aminés reconnaissantes et se liant à un antigène, et
dans laquelle
i) les au moins deux séquences d'acides aminés se liant à l'antigène sont ajoutées à l'extrémité N terminale de la chaîne α et/ou β,
ii) les au moins deux séquences d'acides aminés se liant à l'antigène sont insérées dans une zone constante ou dans une zone variable de ladite chaîne α et/ou β, et/ou
iii) les au moins deux séquences d'acides aminés se liant à l'antigène remplacent certains acides aminés dans une zone constante ou dans une zone variable de ladite chaîne α et/ou β.

2. Protéine de fusion du récepteur de lymphocyte T selon la revendication 1, dans laquelle l'antigène est un antigène de surface cellulaire ou un antigène extracellulaire, de préférence, un antigène spécifique néo-vasculaire.

3. Protéine de fusion du récepteur de lymphocyte T selon les revendications 1 et 2, dans laquelle l'antigène de surface cellulaire est dérivé de l'HER2, de l'ACE (antigène carcino-embryonnaire), ou du PSMA (antigène membranaire spécifique de la prostate).

4. Protéine de fusion du récepteur de lymphocyte T selon une ou plusieurs parmi les revendications précédentes, dans laquelle l'épitope est choisi parmi gp100 ou gp1, ou dans laquelle l'antigène spécifique néo-vasculaire est de l'intégrine αvβ3 ou αvβ5.

5. Protéine de fusion du récepteur de lymphocyte T selon la revendication 1, dans laquelle les au moins deux séquences d'acides aminés se liant à un antigène sont ajoutées à l'extrémité N terminale de la chaîne α et/ou β, et/ou dans laquelle les au moins deux séquences d'acides aminés se liant à un antigène remplacent certains acides aminés dans la boucle de la zone constante de la chaîne β.

6. Protéine de fusion du récepteur de lymphocyte T selon une ou plusieurs parmi les revendications précédentes, dans laquelle ladite protéine de fusion comprend les au moins deux séquences d'acides aminés se liant à un antigène, soit espacées, soit directement en tandem, ou dans laquelle le récepteur de lymphocyte T est exprimé lié à la membrane.

7. Protéine de fusion de chaîne α et/ou β du récepteur de lymphocyte T selon une ou plusieurs parmi les revendications précédentes, comprenant en outre au moins une étiquette d'épitope, dans laquelle ladite étiquette d'épitope est choisie parmi
i) une étiquette d'épitope ajoutée à l'extrémité N terminale et/ou à l'extrémité C terminale de la chaîne α et/ou β,
ii) une étiquette d'épitope insérée dans une zone constante ou variable de ladite chaîne α et/ou β, et
iii) une étiquette d'épitope remplaçant certains acides aminés dans une zone constante ou variable de ladite chaîne α et/ou β.

8. Protéine de fusion du récepteur de lymphocyte T selon une ou plusieurs parmi les revendications précédentes, dans laquelle la séquence d'acide aminé reconnaissant et se liant à un antigène a une longueur entre 5 et 20 acides aminés, de préférence, entre 6 et 12 acides aminés, et dans laquelle la séquence d'acide aminé reconnaissant et se liant à un antigène est de préférence choisie parmi des SEQ ID NO : 1 et/ou 2.

9. Molécule d'acide nucléique isolé codante pour la protéine de fusion du récepteur de lymphocyte T selon une ou plusieurs parmi les revendications précédentes.

10. Molécule d'acide nucléique selon la revendication 9, dans laquelle ladite molécule est choisie parmi l'ADN, de l'ARN, l'ANP (acide nucléique peptidique), l'ANC (acide nucléique circulant), l'ARN messager ou des mélanges de ceux-ci.

11. Vecteur, de préférence un plasmide, un vecteur navette, un phamégide, un cosmide, un vecteur d'expression, un vecteur rétroviral, un vecteur ou une particule adénovirale et/ou un vecteur adapté pour une utilisation en thérapie génique, qui comprend une molécule d'acide nucléique selon les revendications 9 ou 10.

12. Cellule hôte, transfectée avec un vecteur selon la revendication 11 ou un acide nucléique selon la revendication 9, ou infectée et transduite avec une particule selon la revendication 11, ladite cellule étant de préférence une cellule T, une cellule tueuse naturelle, un monocyte, une cellule T tueuse naturelle, une cellule précurseur, une cellule souche hématopoïétique ou une cellule souche non pluripotente, dans laquelle ladite cellule souche exprime une protéine de fusion selon l'une quelconque des revendications 1 à 9 sur sa surface.

13. Procédé d'obtention d'une protéine de fusion selon l'une quelconque des revendications 1 à 8, comprenant une synthèse par voie chimique ou une manipulation génétique dudit peptide, ou comprenant l'expression d'une molécule d'acide nucléique dans une cellule hôte selon la revendication 12, et la purification de ladite protéine de fusion ou dudit récepteur de lymphocyte T à partir de ladite cellule hôte.

14. Composition pharmaceutique, comprenant une protéine de fusion selon l'une quelconque des revendications 1 à 8, une molécule d'acide nucléique selon la revendication 9 ou 10, un vecteur selon la revendication 11 ou une cellule hôte telle que n'importe quelle cellule selon la revendication 12, en association avec un véhicule ou un excipient pharmaceutiquement acceptable.

15. Composition pharmaceutique selon la revendication 14 destinée à une utilisation dans le traitement de tumeurs, de maladies auto-immunes, héréditaires ou infectieuses, de la déficience immunitaire, de l'allergie et/ou pour une utilisation dans le cadre d'une transplantation.
